(19) Europäisches Patentamt
European Patent Office
Office européen des brevets

(11) **EP 2 908 807 B1**

## (12) EUROPEAN PATENT SPECIFICATION

(45) Date of publication and mention
of the grant of the patent:
**25.07.2018 Bulletin 2018/30**

(21) Application number: **12818909.9**

(22) Date of filing: **03.12.2012**

(51) Int Cl.:
*A61N 5/06* (2006.01) *A61F 7/03* (2006.01)
*A61F 7/00* (2006.01) *A61F 7/02* (2006.01)

(86) International application number:
**PCT/IB2012/002569**

(87) International publication number:
**WO 2014/060792 (24.04.2014 Gazette 2014/17)**

(54) **SKIN PATCH FOR ABSORBING TOXINS FROM THE BODY**

HAUTPFLASTER ZUM ABSORBIEREN VON TOXINEN AUS DEM KÖRPER

TIMBRE CUTANÉ DESTINÉ À ABSORBER LES TOXINES DU CORPS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **19.10.2012 US 201213656239**

(43) Date of publication of application:
**26.08.2015 Bulletin 2015/35**

(73) Proprietor: **Salim, Nurman**
**Shimada, Shizuoka 428-0037 (JP)**

(72) Inventor: **Salim, Nurman**
**Shimada, Shizuoka 428-0037 (JP)**

(74) Representative: **Latzel, Klaus**
**Latzel-IP**
**Traunstrasse 3**
**81825 München (DE)**

(56) References cited:
**WO-A1-02/100385 WO-A1-2012/026829**
**US-A1- 2010 121 297 US-A1- 2011 064 787**

Remarks:
The file contains technical information submitted after the application was filed and not included in this specification

**Description**

The present invention relates generally to skin patches. More particularly, the invention relates to a skin patch that absorbs toxins from the body using infrared rays.

Far infrared rays are thought to have the potential to be used to generate blood circulation in the body. In 1870, Dr. Pleasanton, a researcher in the United States, published research showing the relationship between light frequency and the rate of growth of cells and tissues, as well as their rate of cell division. Also, Dr. Neils Finsen, cured lesions and variola using red and infrared rays.

More recent research performed by Tiina Karu, M.D., of the Laser Technology Center in Russia, holds that this spectrum of light speeds up cellular metabolic processes, such as, but not limited to, stimulating the activity of mitochondria, and triggering enzyme activity as well as the healing, regeneration, and normalization of damaged cell tissue. Sunshine is known to emit far infrared rays. Electricity can also generate far infrared rays. A method of far infrared generation with electricity is disclosed in US Patent No. 6,610,082. However, the conversion of sunshine or electricity to generate far infrared rays may not be an efficient method for promoting blood circulation and other cellular processes in the body.

In view of the foregoing, there is a need for improved techniques for providing far infrared rays for use in promoting blood circulation and other cellular processes in the body that do not require sunshine or electricity.

Recently, US 2010/0121297 A1 describes a skin patch for absorbing toxins from a body including a first side having a reflective foil, a second side including a non-woven rayon surface for absorbing toxins, and a mixture of natural ingredients. The mixture is formulated to emit far infrared emission, whereby blood promotion is promoted and any toxins contained therein are absorbed by the rayon surface.

A similar detoxifying patch as the one described before is disclosed in US_2011/0064787 A1. The patch includes ingredients such as herbs and magnetic powder evenly distributed throughout the pouch.

To achieve the forgoing and other objects and in accordance with the purpose of the invention, a skin patch for absorbing toxins from the body is presented in claim 1.

The skin patch according to the invention is specifically adapted to absorb toxins from a body. For this purpose it comprises a first side and a second side. The second side comprises a surface adapted for absorbing and retaining the toxins. The surface comprises a plurality of openings adapted for enhancing transmission of far infrared ray emission into a skin when the second side is placed in contact with the skin.

Between the first side and the second side of the skin patch, a granular mixture of natural ingredients is comprised. The mixture comprises wood vinegar, dextrin, and at least one additional ingredient selected from the group consisting of bamboo vinegar, chitosan, loquat leaf, houttuynia cordata, vitamin C, tourmaline, vegetable fiber, sporopollenin, mushroom chitosan, peppermint, dandelion, blackhaw, fennel, licorice, anethole, glycyrrhizic acid, capsaicin, beta-glucans, pectin, anthocyanins, agaricus, grapefruit, citrus, vaccinum berries, rubus berries, lavender, milk thistle, green tea, epigallocatechin gallate and L-theanine, cinnamon, wasabi, Japanese ginseng, Japanese seaweed, glucomannan, aloe vera, zingerone, shogaol, ginger, gingerol, arnica, nattokinase, winter cherry, beta-elemene, glutathione, holy basil, eucalyptus, amethyst, diatomaceous earth, and zeolite.

The basic components of the natural ingredients are vinegar, for example wood or bamboo vinegar, to inhibit bacterial and fungal growth. The reason is that vinegar has a low concentration of acetic acid in an average range of about 5 to 15 %. In addition to this antibacterial and antifungal action, vinegar is a vasodilator. Vasodilator is a component suitable for widening the opening of the blood vessels and increasing the flow of fluids in the body. This vasodilation effect has been found in case vinegar is taken orally and with skin patches that are applied over a period of time on the skin of a subject to be treated.

Other features, advantages, and objects of the present invention will become more apparent and be more readily understood from the following detailed description of preferred embodiments.

According to a preferred embodiment, a skin patch for advantageously absorbing toxins from a body is presented. The skin patch includes a first side comprising a heat reflective foil and/or a non-woven rayon surface which optionally is covered by a heat-reflective foil. The heat-reflective foil of the skin patch according to this embodiment improves the far infrared absorption of the infrared rays into the skin and, thereby, the vasodilation effect. The foil is a means for reflecting the far infrared rays produced between the first and the second side of the skin patch into the body. More preferably, the heat-reflective foil comprises a metallic foil, such as a titanium or aluminum foil, which optionally comprises carbon particles inside the foil structure and/or sprinkled on the foil surface. Thus, the heat-reflective foil such as a titanium carbon fused foil is comprised of a titanium foil as main component and carbon particles which have been provided, e.g. sprinkled, on top of the titanium foil. The carbon particles preferably are integrally combined ("fused") with the titanium foil on its surface or in its metallic structure.

The exemplified metal component titanium has a good biocompatibility. The carbon particles are advantageous in providing an improved bio-electricity to the skin patch of the invention. In addition, the use of carbon improves the manufacturing process because the carbon particles can lower the humidity level. Humidity is a problem in most food or pharmaceutical packaging as it reduces shelf life to the products. As the ingredients of the skin patches are liable to absorb water, especially if ingredient particles having a significant porosity are used, the use of carbon particles is advantageous. Moreover, carbon removes or lowers odor. This is particularly advantageous if natural ingredients having a significant natural odor are used in the skin patch.

In a further preferred embodiment of the skin patch according to the invention the foil comprises a magnetic material or a number of magnetic cores supported on the foil or compressed between two foils. The magnetic material or cores stipulate the effects of the far-infrared absorption and, thus, improves the detoxification of toxins from the body.

It is preferred that the natural ingredients are sealed between the first side and the second side of the skin patches. Thus, the active material for the detoxification treatment is safely enclosed between the two sides in such a way that the first side functions as a far-infrared ray reflecting surface and the second side which is placed on the skin functions as absorption means for the toxins.

According to a further embodiment of the skin patch of the invention, it is preferred that an adhesive is comprised on a part of the second side for joining the patch to the skin. Joining the patch to the skin means that the adhesive is provided on the whole surface of the second side or at least at the outer edges of the second side of the skin patch to fix the skin patch at the respective place on the body of a subject to be treated.

Alternatively or in addition to the adhesive at the second side, the skin patch according to a further embodiment further optionally comprises means for joining the skin patch to the skin with the second side, wherein the means are provided at the first side. This means can be a strip or a plaster used for sticking the skin patch on the skin of a subject to be treated. These means are suitably adjusted to hold the skin patch during the whole detoxification reaction, which can be a number of hours, for example, 2 to 12 hours, preferably 3 to 10 hours, more preferably, 4 to 8 hours, in particularly bedtime.

In an embodiment of the invention, the at least one additional natural ingredient is prepared from extracting a plant or naturally-occurring substance containing the ingredient with an extraction solvent. After its extraction, the ingredient is contained in the patch in its solid form achieved by removing the extraction solvent or as liquid extract being supported on a solid support structure. Suitable extraction methods are well known to the skilled person.

According to the invention the skin patch contains the natural ingredients in a porous form, that means that at least a part of the natural ingredients are formed as granules having a porous surface. The porous form is advantageous in that toxins can be absorbed not only in the absorbing material of the second side but also within the granules placed between the first and the second side of the skin patch.

It is preferred that the granules having a porous surface have a pore diameter equivalent of less than 30 nm, more preferably about 5 nm to about 25 nm in particular of about 8 to 12 nm, e g. in average about 10 nm. If the shape is not circular, the opening shall have a similar opening area than a circular opening. Therefore, the term "pore diameter equivalent" expresses the diameter or width of the opening regardless of its shape in the form of the value equivalent to the diameter of a circular opening, Depending on the form of the opening, the skilled person knows respective calculations and can easily calculate the respective value.

The advantage of small pores is that the number of pores provided on the granule surface can be increased compared to bigger pores. Thus, the whole active surface for absorbing toxins can be improved, that means increased regarding the identical surface area, with smaller pores.

In order to increase the surface of the granules, it is preferred that the granules have a shape in the form of a capsule or a cigar with a diameter r and a length a. A granule having a spherical form (radius r) has a surface area $A_{Sph}$ according to formula (1):

$$A_{Sph} = 4 * (pi) * r * r \qquad\qquad (1)$$

The surface area of a capsule $A_{Cap}$ having a radius r and a length a is calculated by formula (2):

$$A_{Cap} = 2 * (pi) * r * (2r + a) = A_{Sph} + 2 * (pi) * r * a \qquad (2)$$

The granules with a different shape as a sphere, such as the capsules or cigar-shaped granules have a significantly larger surface as spherical granules. In case the granule size (diameter) cannot be enlarged to increase the surface area, the change of the shape from a sphere to a capsule improves the surface area by the factor 2*(pi)*r*a, which is about 6*r*a. The manufacturing of capsules, that means increasing the side length of the spheres, is easier than manufacturing larger spheres. Therefore, the skin patch according to this embodiment is advantageous in its surface area compared to spherical granules.

According to a further embodiment, the skin patch is adapted to be joined to the skin of a bottom of a foot, that means has the size and shape suitable to be placed as a foot pad. As the skin at the foot is available for the detoxification treatment supported by the natural ingredients and the far-infrared energy provided by the skin patches of the invention, the skin patches are suitable for the application in a detoxification method of a subject by means of placing at least one of the patches at the bottom of the foot for an applicable treatment period. The treatment period is preferably a few hours, more preferably during bedtime.

Advantages of embodiments of the present invention will be apparent from the following detailed description of the exemplary embodiments. The following detailed description should be considered in conjunction with the accompanying figures in which:

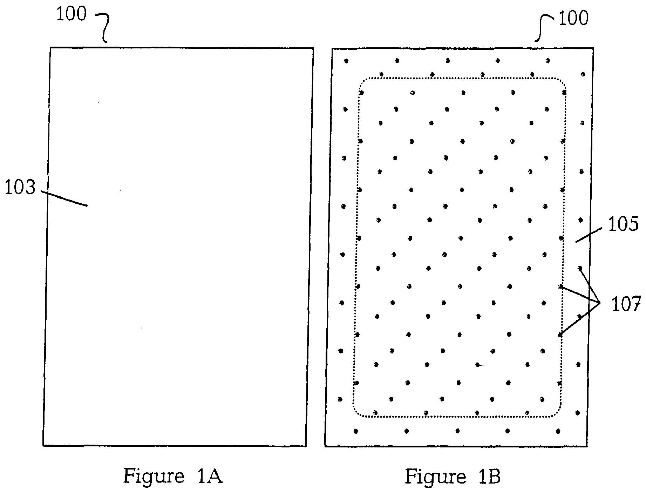

Figures 1A and 1B illustrate an exemplary toxin-absorbing patch, in accordance with an embodiment of the present invention. Figure 1A shows the front of the patch, and Figure 1B shows the back of the patch.

Unless otherwise indicated illustrations in the figures are not necessarily drawn to scale.

Aspects of the invention are disclosed in the following description and related drawings directed to specific embodiments of the invention. Alternate embodiments may be devised without departing from the spirit or the scope of the invention. Additionally, wellknown elements of exemplary embodiments of the invention will not be described in detail or will be omitted so as not to obscure the relevant details of the invention. Further, to facilitate an understanding of the description discussion of several terms used herein follows.

As used herein, the word "exemplary" means "serving as an example, instance or illustration." The embodiments described herein are not limiting, but rather are exemplary only. It should be understood that the described embodiment are not necessarily to be construed as preferred or advantageous over other embodiments. Moreover, the terms "embodiments of the invention", "embodiments" or "invention" do not require that all embodiments of the invention include the discussed feature, advantage or mode of operation.

The present invention is best understood by reference to the detailed figures and description set forth herein. Embodiments of the invention are discussed below with reference to the Figures. However, those skilled in the art will readily appreciate that the detailed description given herein with respect to these figures is for explanatory purposes as the invention extends beyond these limited embodiments. For example, it should be appreciated that those skilled in the art will, in light of the teachings of the present invention, recognize a multiplicity of alternate and suitable approaches, depending upon the needs of the particular application, to implement the functionality of any given detail described herein, beyond the particular implementation choices in the following embodiments described and shown. That is, there are numerous modifications and variations of the invention that are too numerous to be listed but that all fit within the scope of the invention. Also, singular words should be read as plural and vice versa and masculine as feminine and vice versa, where appropriate, and alternative embodiments do not necessarily imply that the two are mutually exclusive. The present invention will now be described in detail with reference to embodiments thereof as illustrated in the accompanying drawings.

Preferred embodiments of the present invention provide a toxin-absorbing patch that is used to generate far infrared rays from natural ingredients without the help of electricity and sunshine. Exemplary purposes of preferred embodiments of the present invention are to help improve blood circulation and to reduce waste and toxins in the body. In a preferred embodiment, a toxin-absorbing patch comprises powdered herbal ingredients. The ingredients are provided in the form of a granular mixture. At least a number, that means at least one, preferably two or more, more preferably all of the ingredients are provided as granules in the form of capsules, for example. The patch is applied to the sole of a foot and emits far infrared rays onto the foot that causes the foot to perspire during application. The warmth from the far infrared rays helps improve blood circulation, and the perspiration absorbed into the patch may contain toxic waste such as, but not limited to, heavy metals. In preferred embodiments, the patch is easy to apply and works in six to eight hours of time. This allows for use during bedtime. In preferred embodiments, adhesive tape or a bandage is used to secure the toxin absorbing patch onto the skin.

Figures 1A and 1B illustrate an exemplary toxin-absorbing patch 100, in accordance with an embodiment of the present invention. Figure 1A shows the front of patch 100, and Figure 1B shows the back of patch 100. The front section of patch 100 is made from thin-layered titanium foil 103, and the rear section of patch 100 is made from non-woven rayon 105. Foil 103 provides a very smooth, shiny and reflective surface, which can reflect heat. Adhesive tape is applied to foil 103 on the front section of patch 100 for application. Rayon 105 on the rear section of patch 100 is applied directly onto the skin during use. Rayon 105 comprises openings 107 that offer enhanced far infrared ray emission into the skin. Patch 100 preferably has a width of 60 mm and a length of 80 to 90 mm; however, patches in alternate embodiments may be various shapes and sizes. Patch 100 has large surface area so that it can cover a large section of the body. The weight of patch 100 is preferably five grams with an error level of 0.2 grams; however, patches in alternate embodiments may weigh more or less.

In other exemplary embodiments, the front section of patch 100 may be made from non-woven rayon, and the rear section of patch 100 may also be made from nonwoven rayon. In yet other exemplary embodiments, the front section of patch 100 may be made from thin-layered titanium foil, and the rear section of patch 100 may also be made from thin layered titanium foil. The titanium foil is, in this exemplary embodiment, fused with carbon, i. e. carbon particles are provided on the surface of the titanium foil. In yet other exemplary embodiments, the front and rear sections of patch 100 may be made from any suitable materials that enable patch 100 to function as described herein.

In the exemplary embodiment, powdered natural ingredients are sealed inside patch 100 between foil 103 and rayon 105. Patch 100 includes a sachet between foil 103 and rayon 105 that has a bag opening into which the natural ingredients such as, but not limited to, vinegar, chitosan, loquat leaf, houttuynia cordata, vitamin C, tourmaline, vegetable fiber, and dextrin may be inserted. In the present embodiment, the bag section of the sachet is large and can be made to contain five to eight grams of ingredients. This mixture of natural ingredients is formulated to emit heat, or far infrared rays. Table 1 lists an exemplary formula for the natural ingredients in a toxin-absorbing patch, according to an embodiment of the present invention. Those skilled in the art, in light of the present teachings, will readily recognize that various other types of ingredients and amounts of ingredients may be used in alternate embodiments.

In typical use of the present embodiment, patch 100 is applied externally on the surface of the skin. Patch 100 is attached to a bandage or adhesive tape before application to the skin. A user applies patch 100 to the skin by joining patch 100 to the bandage or adhesive tape with the edges of the bandage or adhesive tape extending beyond the edges of patch 100 and then attaching patch 100 with the adhesive tape or bandage to any location on the body that requires far infrared rays. Typically, the user applies patch 100 onto the bottom of a foot for six to eight hours. This allows for use during bedtime. During use, the foot produces sweat due to the heat coming from patch 100, and the sweat may include toxins such as, but not limited to, heavy metals, which are absorbed into patch 100. Therefore, a user that is suffering from heavy metal toxicity, for example, without limitation, a person with Autism, may benefit from the use of patch 100 because of the heavy metal reduction capability. The warmth from the far infrared rays also may help to improve blood circulation, and people suffering from blocked blood circulation may benefit from using patch 100. In the present embodiment, patch 100 fits virtually any part of the body, is easy to use, and generally eliminates the need for electrical devices and interference. Patch 100 is made of non-allergic material, and may therefore be used by people who have allergies.

Testing has shown that patches according to embodiments of the present invention may be used to emit heat when comprising a correct mixture of natural ingredients. Previously, only devices using electrical currents could be used to generate far infrared emission. Furthermore, an analysis of a new toxin-absorbing patch and a used toxin-absorbing patch was performed by Japan Food Research Laboratories on December 2, 2005. The analysis tested for various heavy metals in the two patches including arsenic, lead, cadmium, mercury, tin, cobalt, and chromium. In the analysis only 0.01 parts per million (ppm) of cadmium was detected and none of the other metals were detected in the new patch while 0.55 ppm of lead, 0.03 ppm of cadmium, 0.09 ppm of cobalt, and 1.7 ppm of chromium were detected in the used patch. This analysis provides evidence that embodiments of the present invention may be used for the absorption of heavy metal from the body into the patch.

Additional exemplary embodiments of the patch 100 described herein may include formulations having a variety of ingredients in diverse combinations. The ingredients for the formulations may be selected from the non-limiting list of: loquat leaf (Eriobotrya japonica); dokudami (Houttuynia cordata); peppermint (Mentha piperita); dandelion (Taraxacum officinale); blackhaw (Viburnum prunifolium); fennel (Foeniculum vulgare); licorice (Glycyrrhiza glabra); agaricus; grapefruit (Citrus × paradisi); raspberry (Rubus); lavender (Lavandula); milk thistle (Silybum marianum); cinnamon (Cinnamomum); japanese ginseng (Panax japonicus); japanese seaweed (kaiso); konjac (Amorphophallus konjac); aloe vera; ginger (Zingiber officinale); arnica, which may be an arnica species not containing helenalin; winter cherry (ashwagandha or Withania somnifera); holy basil (tulsi or Ocimum tenuiflorum); wasabi (Wasabia japonica); eucalyptus; Vitamin C; wood vinegar; bamboo vinegar; vegetable fiber; glycyrrhizic acid, beta-glucans; sporopollenin; glucomannan; anethole, mushroom chitosan; chitosan; pectin, anthocyanins; zingerone; shogaol; gingerol; dextrin; capsaicin; betaelemene; glutathione; Epigallocatechin gallate (EGCG) and L-Theanine; nattokinase; tourmaline; amethyst; diatomaceous earth; and zeolite.

In some exemplary embodiments of the formulations, certain active ingredients listed above may be extracted from a particular plant or naturally-occurring substance containing the active ingredient. For example, sporopollenin may be extracted from Chlorella vulgaris; capsaicin may be extracted from capsicum peppers; anthocyanins may be extracted from Vaccinum berries, Rubus berries, or the like; EGCG and L-Theanine may be extracted from green tea; dextrin may be extracted from cornstarch; glycyrrhizic acid may be extracted from licorice; beta-glucans may be extracted from agaricus; pectin may be extracted from grapefruit, citrus fruits, or any other suitable plant; anethole may be extracted from fennel; glucomannan may be extracted from konjac; zingerone, shogaol, and gingerol may be extracted from ginger; beta-elemene and glutathione may be extracted from holy basil or any other suitable plant, and so forth. However, such active ingredients may be extracted from any suitable plant species containing the ingredient and included in the formulation.

It should be appreciated that some exemplary embodiments of the formulations may include the above-described active ingredients as extracted from the plant matter containing the active ingredient. In other exemplary embodiments of the formulations, the active ingredients may be included in the formulations as part of the plant matter containing the active ingredients. It should further be appreciated that the amount of active ingredient in a particular plant may be an amount less than the total composition of the particular plant. For example, glycyrrhizic acid may be about 64% of licorice, by weight; beta-glucans may be about 99% of agaricus by weight; pectin may be about 33% of grapefruit by weight; EGCG may be about 80% of green tea by weight; L-theanine may be about 19% of green tea by weight; and glucomannan may be about 99% of konjac-mannan by weight. Therefore, where the below listed formulations recite a particular plant, the formulations should also be understood as encompassing any formulation containing any active ingredient of the plant, with the percentage by weight of the active ingredient in the formulation being substantially similar to the percentage by weight of the plant multiplied by the percentage by weight of the active ingredient contained in the plant. The formulations listed below should also be understood as equivalent to formulations including any substance, such as plant matter or the like, that includes the particular active ingredient.

Various embodiments of formulations for patch 100 may include the above-described ingredients in varying proportions. As shown in Table 1, a particular formulation may contain between 0% to 100%, by weight, of each of the above-described ingredients. It should be appreciated that the percentage by weight of each ingredient of the formulation may be adjusted as desired within the given weight-percent range.

Table 1

| Ingredient | Wt. % |
| --- | --- |
| Wood Vinegar | 0%-100% |
| Bamboo Vinegar | 0%-100% |
| Chitosan | 0%-100% |
| Loquat Leaf | 0%-100% |
| Houttuynia cordata | 0%-100% |
| Vitamin C | 0%-100% |
| Tourmaline | 0%-100% |
| Dextrin | 0%-100% |
| Vegetable fiber | 0%-100% |
| Sporopollenin | 0%-100% |
| Mushroom Chitosan | 0%-100% |
| Peppermint | 0%-100% |
| Dandelion | 0%-100% |
| Blackhaw | 0%-100% |
| Fennel | 0%-100% |
| Licorice | 0%-100% |
| Capsaicin | 0%-100% |
| Agaricus | 0%-100% |
| Grapefruit/citrus | 0%-100% |
| Vaccinum/Rubus berries | 0%-100% |
| Lavender | 0%-100% |
| Milk Thistle | 0%-100% |
| Green tea | 0%-100% |
| Cinnamon | 0%-100% |
| Wasabi | 0%-100% |
| Japanese ginseng | 0%-100% |
| Japanese seaweed | 0%-100% |
| Glucomannan | 0%-100% |
| Aloe Vera | 0%-100% |
| Ginger | 0%-100% |
| Arnica | 0%-100% |
| Nattokinase | 0%-100% |
| Winter cherry | 0%-100% |
| Holy basil | 0%-100% |
| Eucalyptus | 0%-100% |

(continued)

| Ingredient | Wt. % |
|---|---|
| Amethyst | 0%-100% |
| Diatomaceous earth | 0%-100% |
| Zeolite | 0%-100% |

Exemplary embodiments of patch 100 may have total weights in the range between about 3 grams and about 50 grams. The embodiments of patch 100 may be provided within this range, for example, in 1 gram increments. Therefore, exemplary embodiments of patch 100 may include patches having a weight of 3 g, 4 g, 5 g, ... , 48 g, 49 g, 50g, inclusive of all intermediate sizes.

Additionally, patch 100 may be provided in any desired shape or size. Exemplary embodiments of patch 100 may have a length in the range between about 100 mm to about 2000 mm, and a width in the range between about 100 mm to about 2000 mm. Any sizes of patch 100 within these ranges may be provided, as a non-limiting example, 600 mm × 800 mm, 600 mm × 900 mm, 600 mm × 1000 mm, 600 mm × 1100 mm, 500 mm × 800 mm, and so forth. Patch 100 may be shaped as a circle, triangle, quadrilateral, or any other desired shape. In some exemplary embodiments, patch 100 may have a radius between about 100 mm to about 2000 mm. Therefore, any radii for patch 100 within these ranges may be provided, for example a radius of 100 mm, 200 mm, 300 mm, ... , 1800 mm, 1900 mm, 2000 mm, inclusive of all intermediate sizes.

Tables 2-38 illustrate additional exemplary embodiments of formulations for patch 100. The ingredient proportions in the listed formulations should be considered exemplary and not limiting. It should also be appreciated that, for each composition, the percentage by weight of each ingredient may vary by approximately 50% of the indicated percentage by weight value for that ingredient. The formulations listed herein, as well as the percentage by weight values of the ingredients therein, may be applicable to embodiments of patch 100 having any desired weight or shape.

Table 2

| Ingredient | Wt. % |
|---|---|
| Wood Vinegar | 12.00%±6.00% |
| Bamboo Vinegar | 12.00%±6.00% |
| Chitosan | 0.80%±0.40% |
| Loquat Leaf | 0.80%±0.40% |
| Houttuynia cordata | 0.80%±0.40% |
| Vitamin C | 0.80%±0.40% |
| Tourmaline | 60.00%±30.00% |
| Vegetable Fiber | 4.00%±2.00% |
| Dextrin | 8.80%±4.40% |

Table 3

| Ingredient | Wt. % |
|---|---|
| Wood Vinegar | 26.00%±13.00% |
| Bamboo Vinegar | 15.00%±7.50% |
| Chitosan | 1.00%±0.50% |
| Loquat Leaf | 1.00%±0.50% |
| Houttuynia cordata | 5.00%±2.50% |
| Vitamin C | 2.00%±1.00% |
| Tourmaline | 30.00%±15.00% |
| Dextrin | 20.00%±10.00% |

Table 4

| Ingredient | Wt. % |
|---|---|
| Wood Vinegar | 15.00%±7.50% |
| Bamboo Vinegar | 15.00%±7.50% |
| Chitosan | 1.00%±0.50% |
| Loquat Leaf | 1.00%±0.50% |
| Houttuynia cordata | 1.00%±0.50% |
| Vitamin C | 1.00%±0.50% |
| Tourmaline | 50.00%±25.00% |
| Dextrin | 11.00%±5.50% |
| Vegetable fiber | 5.00%±2.50% |

Table 5

| Ingredient | Wt. % |
|---|---|
| Wood Vinegar | 11.76%±5.88% |
| Bamboo Vinegar | 7.06±3.53%% |
| Loquat Leaf | 0.47%±0.235% |
| Houttuynia cordata | 0.47%±0.235% |
| Vitamin C | 0.47%±0.235% |
| Tourmaline | 70.59%±35.295% |
| Dextrin | 1.18%±0.59% |
| Vegetable fiber | 2.35%±1.175% |
| Sporopollenin | 5.18%±0.259% |
| Mushroom Chitosan | 0.47%±0.235% |

Table 6

| Ingredient | Wt. % |
|---|---|
| Wood Vinegar | 20.00%±10.00% |
| Tourmaline | 40.00%±20.00% |
| Dextrin | 4.00%±2.00% |
| Vegetable fiber | 4.00%±2.00% |
| Peppermint | 16.00%±8.00% |
| Dandelion | 4.00%±2.00% |
| Blackhaw | 4.00%±2.00% |
| Fennel | 4.00%±2.00% |
| Licorice | 4.00%±2.00% |

Table 7

| Ingredient | Wt. % |
|---|---|
| Wood Vinegar | 20.00%±10.00% |
| Tourmaline | 50.00%±25.00% |
| Dextrin | 2.00%±1.00% |
| Peppermint | 20.00%±10.00% |
| Dandelion | 2.00%±1.00% |
| Blackhaw | 2.00%±1.00% |
| Fennel | 2.00%±1.00% |
| Licorice | 2.00%±1.00% |

Table 8

| Ingredient | Wt. % |
|---|---|
| Wood Vinegar | 25.00%±12.50% |
| Bamboo Vinegar | 25.00%±12.50% |
| Chitosan | 0.50%±0.25% |
| Loquat Leaf | 5.00%±2.50% |
| Houttuynia cordata | 5.00%±2.50% |
| Vitamin C | 2.00%±1.00% |
| Tourmaline | 18.75%±9.375% |
| Dextrin | 12.50%±6.25% |
| Amethyst | 6.25%±3.125% |

Table 9

| Ingredient | Wt. % |
|---|---|
| Wood Vinegar | 23.75%±11.875% |
| Bamboo Vinegar | 56.25%±28.125% |
| Chitosan | 1.00%±0.50% |
| Loquat Leaf | 1.00%±0.50% |
| Houttuynia cordata | 2.00%±1.00% |
| Vitamin C | 1.00%±0.50% |
| Dextrin | 15.00%±7.50% |

Table 10

| Ingredient | Wt. % |
|---|---|
| Wood Vinegar | 25.00%±12.50% |
| Bamboo Vinegar | 56.25%±28.125% |
| Chitosan | 1.00%±0.50% |
| Loquat Leaf | 1.00%±0.50% |

(continued)

| Ingredient | Wt. % |
| --- | --- |
| Houttuynia cordata | 2.00%±1.00% |
| Vitamin C | 1.00%±0.50% |
| Dextrin | 13.75%±6.875% |

Table 11

| Ingredient | Wt. % |
| --- | --- |
| Wood Vinegar | 15.00%±12.50% |
| Bamboo Vinegar | 15.00%±12.50% |
| Chitosan | 1.00%±0.50% |
| Loquat Leaf | 1.00%±0.50% |
| Houttuynia cordata | 1.50%±0.75% |
| Vitamin C | 1.00%±0.50% |
| Capsaicin | 2.50%±1.25% |
| Vegetable Fiber | 20.00%±10.00% |
| Dextrin | 43.00%±21.50% |

Table 12

| Ingredient | Wt. % |
| --- | --- |
| Wood Vinegar | 15.00%±7.50% |
| Bamboo Vinegar | 25.00%±12.50% |
| Chitosan | 1.00%±0.50% |
| Loquat Leaf | 1.00%±0.50% |
| Houttuynia cordata | 1.50%±0.75% |
| Vitamin C | 1.00%±0.50% |
| Capsaicin | 5.00%±2.50% |
| Vegetable Fiber | 20.00%±10.00% |
| Dextrin | 30.50%±15.25% |

Table 13

| Ingredient | Wt. % |
| --- | --- |
| Wood Vinegar | 16.00%±8.00% |
| Bamboo Vinegar | 20.00%±10.00% |
| Chitosan | 0.04%±0.02% |
| Loquat Leaf | 0.04%±0.02% |
| Houttuynia cordata | 0.06%±0.03% |
| Vitamin C | 0.80%±0.40% |
| Capsaicin | 1.00%±0.50% |

(continued)

| Ingredient | Wt. % |
|---|---|
| Vegetable Fiber | 16.00%±8.00% |
| Dextrin | 24.40%±12.20% |

Table 14

| Ingredient | Wt. % |
|---|---|
| Wood Vinegar | 15.00%±7.50% |
| Bamboo Vinegar | 15.00%±7.50% |
| Tourmaline | 5.00%±2.50% |
| Agaricus | 5.00%±2.50% |
| Dextrin | 60.00%±30.00% |

Table 15

| Ingredient | Wt. % |
|---|---|
| Wood Vinegar | 12.00%±6.00% |
| Bamboo Vinegar | 12.00%±6.00% |
| Tourmaline | 20.00%±10.00% |
| Agaricus | 8.00%±4.00% |
| Dextrin | 48.00%±24.00% |

Table 16

| Ingredient | Wt. % |
|---|---|
| Wood Vinegar | 15.00%±7.50% |
| Bamboo Vinegar | 15.00%±7.50% |
| Vitamin C | 1.00%±0.50% |
| Vegetable Fiber | 20.00%±10.00% |
| Dextrin | 49.00%±24.50% |

Table 17

| Ingredient | Wt. % |
|---|---|
| Wood Vinegar | 16.00%±8.00% |
| Bamboo Vinegar | 16.00%±8.00% |
| Vitamin C | 4.80%±2.40% |
| Vegetable Fiber | 20.00%±10.00% |
| Dextrin | 43.20%±21.60% |

Table 18

| Ingredient | Wt. % |
|---|---|
| Wood Vinegar | 15.00%±7.50% |
| Bamboo Vinegar | 56.25%±28.125% |
| Loquat Leaf | 1.00%±0.50% |
| Houttuynia cordata | 2.00%±1.00% |
| Grapefruit/citrus | 2.00%±1.00% |
| Dextrin | 23.75%±11.875% |

Table 19

| Ingredient | Wt. % |
|---|---|
| Wood Vinegar | 16.00%±8.00% |
| Bamboo Vinegar | 45.00%±22.50% |
| Loquat Leaf | 2.00%±1.00% |
| Houttuynia cordata | 2.00%±1.00% |
| Grapefruit/citrus | 16.00%±8.00% |
| Dextrin | 19.00%±9.50% |

Table 20

| Ingredient | Wt. % |
|---|---|
| Wood Vinegar | 15.00%±7.50% |
| Bamboo Vinegar | 56.25%±28.125% |
| Loquat Leaf | 1.00%±0.50% |
| Houttuynia cordata | 2.00%±1.00% |
| Vaccinum/Rubus berries | 2.00%±1.00% |
| Dextrin | 23.75%±11.275% |

Table 21

| Ingredient | Wt. % |
|---|---|
| Wood Vinegar | 16.00%±8.00% |
| Bamboo Vinegar | 45.00%±22.50% |
| Loquat Leaf | 2.00%±1.00% |
| Houttuynia cordata | 2.00%±1.00% |
| Vaccinum/Rubus berries | 16.00%±8.00% |
| Dextrin | 19.00%±9.50% |

Table 22

| Ingredient | Wt. % |
|---|---|
| Wood Vinegar | 15.00%±7.50% |
| Bamboo Vinegar | 56.25%±28.125% |
| Loquat Leaf | 1.00%±0.50% |
| Houttuynia cordata | 2.00%±1.00% |
| Lavender | 2.00%±1.00% |
| Dextrin | 23.75%±11.275% |

Table 23

| Ingredient | Wt. % |
|---|---|
| Wood Vinegar | 16.00%±8.00% |
| Bamboo Vinegar | 45.00%±22.50% |
| Loquat Leaf | 0.80%±0.40% |
| Houttuynia cordata | 1.60%±0.80% |
| Tourmaline | 5.60%±2.80% |
| Lavender | 20.00%±10.00% |
| Dextrin | 11.00%±5.50% |

Table 24

| Ingredient | Wt. % |
|---|---|
| Wood Vinegar | 25.00%±12.50% |
| Bamboo Vinegar | 25.00%±12.50% |
| Loquat Leaf | 1.50%±0.75% |
| Houttuynia cordata | 1.00%±0.50% |
| Tourmaline | 12.50%±6.25% |
| Grapefruit/citrus | 25.00%±12.50% |
| Dextrin | 10.00%±5.00% |

Table 25

| Ingredient | Wt. % |
|---|---|
| Wood Vinegar | 20.00%±10.00% |
| Bamboo Vinegar | 20.00%±10.00% |
| Loquat Leaf | 1.20%±0.60% |
| Houttuynia cordata | 0.80%±0.40% |
| Tourmaline | 20.00%±10.00% |
| Grapefruit/citrus | 30.00%±15.00% |
| Dextrin | 8.00%±4.00% |

Table 26

| Ingredient | Wt. % |
|---|---|
| Wood Vinegar | 25.00%±12.50% |
| Bamboo Vinegar | 12.50%±6.25% |
| Loquat Leaf | 5.00%±2.50% |
| Houttuynia cordata | 5.00%±2.50% |
| Tourmaline | 12.50%±6.25% |
| Vegetable Fiber | 5.00%±2.50% |
| Capsaicin | 5.00%±2.50% |
| Milk thistle | 10.00%±5.00% |
| Dextrin | 20.00%±10.00% |

Table 27

| Ingredient | Wt. % |
|---|---|
| Wood Vinegar | 15.00%±7.50% |
| Bamboo Vinegar | 15.00%±7.50% |
| Loquat Leaf | 0.50%±0.25% |
| Houttuynia cordata | 0.50%±0.25% |
| Vitamin C | 0.50%±0.25% |
| Tourmaline | 12.50%±6.25% |
| Vegetable Fiber | 1.00%±0.50% |
| Green tea | 50.00%±25.00% |
| Dextrin | 5.00%±2.50% |

Table 28

| Ingredient | Wt. % |
|---|---|
| Wood Vinegar | 12.00%±6.00% |
| Bamboo Vinegar | 12.00%±6.00% |
| Loquat Leaf | 0.40%±0.20% |
| Houttuynia cordata | 0.40%±0.20% |
| Vitamin C | 0.40%±0.20% |
| Tourmaline | 20.00%±10.00% |
| Vegetable Fiber | 0.80%±0.40% |
| Green tea | 50.00%±25.00% |
| Dextrin | 4.00%±2.00% |

Table 29

| Ingredient | Wt. % |
|---|---|
| Wood Vinegar | 15.00%±7.50% |
| Bamboo Vinegar | 15.00%±7.50% |
| Chitosan | 0.50%±0.25% |
| Loquat Leaf | 0.50%±0.25% |
| Houttuynia cordata | 0.50%±0.25% |
| Tourmaline | 37.50%±18.75% |
| Vegetable Fiber | 0.50%±0.25% |
| Cinnamon | 10.00%±5.00% |
| Japanese ginseng | 5.00%±2.50% |
| Japanese seaweed | 5.00%±2.50% |
| Glucomannan | 5.00%±2.50% |
| Dextrin | 5.50%±2.50% |

Table 30

| Ingredient | Wt. % |
|---|---|
| Wood Vinegar | 12.00%±6.00% |
| Bamboo Vinegar | 12.00%±6.00% |
| Chitosan | 0.40%±0.20% |
| Loquat Leaf | 0.40%±0.20% |
| Houttuynia cordata | 0.40%±0.20% |
| Tourmaline | 50.00%±25.00% |
| Vegetable Fiber | 0.40%±0.20% |
| Cinnamon | 8.00%±4.00% |
| Japanese ginseng | 4.00%±2.00% |
| Japanese seaweed | 4.00%±2.00% |
| Glucomannan | 4.00%±2.00% |
| Dextrin | 4.40%±2.00% |

Table 31

| Ingredient | Wt. % |
|---|---|
| Wood Vinegar | 12.00%±6.00% |
| Bamboo Vinegar | 8.00%±4.00% |
| Houttuynia cordata | 4.00%±2.00% |
| Vitamin C | 0.80%±0.40% |
| Tourmaline | 20.00%±10.00% |
| Vegetable Fiber | 2.00%±1.00% |
| Diatomaceous earth | 20.00%±10.00% |

(continued)

| Ingredient | Wt. % |
|---|---|
| Aloe Vera | 2.04%±1.02% |
| Ginger | 1.98%±0.99% |
| Arnica | 1.98%±0.99% |
| Zeolite | 20.00%±10.00% |
| Dextrin | 7.20%±3.60% |

Table 32

| Ingredient | Wt. % |
|---|---|
| Wood Vinegar | 11.76%±5.88% |
| Bamboo Vinegar | 11.76%±5.88% |
| Loquat Leaf | 0.47%±0.235% |
| Houttuynia cordata | 0.47%±0.235% |
| Vitamin C | 0.94%±0.47% |
| Tourmaline | 35.29%±17.645% |
| Vegetable Fiber | 2.35%±1.175% |
| Mushroom chitosan | 0.47%±0.235% |
| Nattokinase | 11.76%±5.88% |
| Winter cherry | 11.76%±5.88% |
| Holy basil | 11.76%±5.88% |
| Dextrin | 1.18%±0.59% |

Table 33

| Ingredient | Wt. % |
|---|---|
| Wood Vinegar | 8.00%±4.00% |
| Bamboo Vinegar | 12.00%±6.00% |
| Chitosan | 16.00%±8.00% |
| Tourmaline | 30.00%±15.00% |
| Agaricus | 4.00%±2.00% |
| Eucalyptus | 10.00%±5.00% |
| Dextrin | 20.00%±10.00% |

Table 34

| Ingredient | Wt. % |
|---|---|
| Wood Vinegar | 7.06%±3.53% |
| Bamboo Vinegar | 7.06%±3.53% |
| Loquat Leaf | 0.24%±0.12% |
| Houttuynia cordata | 0.24%±0.12% |

(continued)

| Ingredient | Wt. % |
|---|---|
| Tourmaline | 70.59%±35.295% |
| Sporopollenin | 2.94%±1.47% |
| Amethyst | 2.94%±1.47% |
| Agaricus | 2.94%±1.47% |
| Zeolite | 2.94%±1.47% |
| Dextrin | 0.26%±0.13% |

Table 35

| Ingredient | Wt. % |
|---|---|
| Wood Vinegar | 11.76%±5.88% |
| Bamboo Vinegar | 7.06%±3.53% |
| Loquat Leaf | 0.24%±0.12% |
| Houttuynia cordata | 0.24%±0.12% |
| Vitamin C | 0.24%±0.12% |
| Tourmaline | 70.59%±35.295% |
| Vegetable fiber | 1.18±0.59% |
| Sporopollenin | 7.06%±3.53% |
| Mushroom chitosan | 0.47%±0.235% |
| Dextrin | 0.18%±0.09% |

Table 36

| Ingredient | Wt. % |
|---|---|
| Wood Vinegar | 10.00%±5.00% |
| Bamboo Vinegar | 6.00%±3.00% |
| Loquat Leaf | 0.20%±0.10% |
| Houttuynia cordata | 0.20%±0.10% |
| Vitamin C | 0.20%±0.10% |
| Tourmaline | 70.00%±35.00% |
| Vegetable fiber | 2.00±1.00% |
| Sporopollenin | 10.00%±5.00% |
| Mushroom chitosan | 0.40%±0.20% |
| Dextrin | 1.00%±0.50% |

Table 37

| Ingredient | Wt. % |
|---|---|
| Wood Vinegar | 9.09%±4.545% |
| Bamboo Vinegar | 5.45%±2.725% |

(continued)

| Ingredient | Wt. % |
|---|---|
| Loquat Leaf | 0.18%±0.09% |
| Houttuynia cordata | 0.18%±0.09% |
| Vitamin C | 0.18%±0.09% |
| Tourmaline | 72.73%±36.365% |
| Vegetable fiber | 1.82±0.91% |
| Sporopollenin | 9.09%±4.545% |
| Mushroom chitosan | 0.36%±0.18% |
| Dextrin | 0.91%±0.455% |

Table 38

| Ingredient | Wt. % |
|---|---|
| Wood Vinegar | 20.00%±10.00% |
| Bamboo Vinegar | 45.00%±22.50% |
| Chitosan | 0.80%±0.40 |
| Loquat Leaf | 0.80%±0.40% |
| Houttuynia cordata | 1.60%±0.80% |
| Wasabi | 20.00%±10.00% |
| Vegetable fiber | 2.00±1.00% |
| Dextrin | 11.00%±5.50% |

Having fully described at least one embodiment of the present invention, other equivalent or alternative methods of providing toxin absorption and improving blood circulation using far infrared rays according to the present invention will be apparent to those skilled in the art. The invention has been described above by way of illustration, and the specific embodiments disclosed are not intended to limit the invention to the particular forms disclosed. For example, the particular implementation of the patch may vary depending upon the intended location of application. The patches described in the foregoing were directed to implementations typically applied to the bottom of the foot; however, similar techniques are to provide patches that are shaped to specifically fit on various parts of the body. For example, without limitation, large sheets may be used on the back or the back of the legs, curved patches may be used on the underarms, and small, round patches may be used on the hands. Implementations of the present invention that are applied to various areas of the body are contemplated as within the scope of the present invention. The invention is thus to cover all modifications, equivalents, and alternatives falling within the spirit and scope of the following claims.

The foregoing description and accompanying figures illustrate the principles, preferred embodiments and modes of operation of the invention. However, the invention should not be construed as being limited to the particular embodiments discussed above. Additional variations of the embodiments discussed above will be appreciated by those skilled in the art.

Therefore, the above-described embodiments should be regarded as illustrative rather than restrictive. Accordingly, it should be appreciated that variations to those embodiments can be made by those skilled in the art without departing from the scope of the invention as defined by the following claims and can particularly in line with the following aspects:

1. A skin patch for absorbing toxins from a body, comprising:

a first side;
a second side comprising a surface configured for absorbing and retaining the toxins, the surface comprising a plurality of openings configured for enhancing transmission of far infrared ray emission into a skin when the second side is placed in contact with the skin; and

a mixture of natural ingredients, the mixture comprising wood vinegar, dextrin, and at least one additional ingredient selected from the group consisting of:

bamboo vinegar, chitosan, loquat leaf, houttuynia cordata, vitamin C, tourmaline, vegetable fiber, sporopollenin, mushroom chitosan, peppermint, dandelion, blackhaw, fennel, licorice, capsaicin, agaricus, grapefruit, citrus, vaccinum berries, rubus berries, lavender, milk thistle, green tea, cinnamon, wasabi, Japanese ginseng, Japanese seaweed, glucomannan, aloe vera, ginger, arnica, nattokinase, winter cherry, holy basil, eucalyptus, amethyst, diatomaceous earth, and zeolite.

2. The skin patch of aspect 1, wherein the first side is formed from one of a heatreflective foil or a non-woven rayon surface.

3. The skin patch of aspect 1, wherein the second side is formed from one of a heatreflective foil or a non-woven rayon surface.

4. The skin patch of aspect 1, wherein the natural ingredients are sealed between the first side and the second side.

5. The skin patch of aspect 1, wherein the skin patch is operable to be joined to the skin with an adhesive.

6. The skin patch of aspect 1, wherein the skin patch is operable to be joined to the skin of a bottom of a foot.

7. A skin patch for absorbing toxins from a body, comprising:

a first side;

a second side comprising a surface configured for absorbing and retaining the toxins, the surface comprising a plurality of openings configured for enhancing transmission of far infrared ray emission into a skin when the second side is placed in contact with the skin; and

a mixture of natural ingredients, the mixture comprising wood vinegar, dextrin, and at least one additional ingredient selected from the group consisting of: bamboo vinegar, chitosan, loquat leaf, houttuynia cordata, vitamin C, tourmaline, vegetable fiber, sporopollenin, mushroom chitosan, peppermint, dandelion, blackhaw, anethole, glycyrrhizic acid, capsaicin, beta-glucans, pectin, anthocyanins, lavender, milk thistle, EGCG and Ltheanine, cinnamon, wasabi, Japanese ginseng, Japanese seaweed, glucomannan, aloe vera, zingerone, shogaol, gingerol, arnica, nattokinase, winter cherry, beta-elemene, glutathione, eucalyptus, amethyst, diatomaceous earth, and zeolite.

8. The skin patch of aspect 7, wherein the first side is formed from one of a heatreflective foil or a non-woven rayon surface.

9. The skin patch of aspect 7, wherein the second side is formed from one of a heatreflective foil or a non-woven rayon surface.

10. The skin patch of aspect 7, wherein the natural ingredients are sealed between the first side and the second side.

11. The skin patch of aspect 7, wherein the skin patch is operable to be joined to the skin with an adhesive.

12. The skin patch of aspect 7, wherein the skin patch is operable to be joined to the skin of a bottom of a foot.

13. A skin patch for absorbing toxins from a body, comprising:

means for reflecting heat from far infrared ray emissions;

means for absorbing and retaining the toxins;

means for enhancing transmission of far infrared ray emissions into a skin; and

means for emitting said far infrared ray emissions, whereby blood circulation is promoted and perspiration and any toxins contained therein are absorbed by said absorbing means;

wherein the skin patch contains a mixture of natural ingredients, the mixture comprising wood vinegar, dextrin, and at least one additional ingredient selected from the group consisting of:

bamboo vinegar, chitosan, loquat leaf, houttuynia cordata, vitamin C, tourmaline, vegetable fiber, sporopollenin, mushroom chitosan, peppermint, dandelion, blackhaw, fennel, licorice, capsaicin, agaricus, grapefruit, citrus, vaccinum berries, rubus berries, lavender, milk thistle, green tea, cinnamon, wasabi, Japanese ginseng, Japanese seaweed, glucomannan, aloe vera, ginger, arnica, nattokinase, winter cherry, holy basil, eucalyptus, amethyst, diatomaceous earth, and zeolite.

14. The skin patch of aspect 13, further comprising means for joining the skin patch to the skin.

**Claims**

1. A skin patch for absorbing toxins from a body, comprising:

   a first side;
   a second side comprising a surface adapted for absorbing and retaining the toxins, the surface comprising a plurality of openings adapted for enhancing transmission of far infrared ray emission into a skin when the second side is placed in contact with the skin; and
   a granular mixture of natural ingredients, wherein at least a part of the natural ingredients are formed as granules having a porous surface and the mixture comprises wood vinegar, dextrin, and at least one additional ingredient selected from the group consisting of:
   bamboo vinegar, chitosan, loquat leaf, houttuynia cordata, vitamin C, tourmaline, vegetable fiber, sporopollenin, mushroom chitosan, peppermint, dandelion, blackhaw, fennel, licorice, anethole, glycyrrhizic acid, capsaicin, beta-glucans, pectin, anthocyanins, agaricus, grapefruit, citrus, vaccinum berries, rubus berries, lavender, milk thistle, green tea, epigallocatechin gallate and L-theanine, cinnamon, wasabi, Japanese ginseng, Japanese seaweed, glucomannan, aloe vera, zingerone, shogaol, ginger, gingerol, arnica, nattokinase, winter cherry, beta-elemene, glutathione, holy basil, eucalyptus, amethyst, diatomaceous earth, and zeolite.

2. The skin patch of claim 1, wherein the first side comprises a heat-reflective foil and/or a non-woven rayon surface which optionally is covered by a heat-reflective foil.

3. The skin patch of claim 1 or claim 2, wherein the heat-reflective foil comprises a metallic foil comprising carbon particles in its foil structure and/or sprinkled on the foil surface.

4. The skin patch according to any of claims 1 to 3, wherein the foil comprises a magnetic material or a number of magnetic cores supported on a foil or compressed between two foils.

5. The skin patch of anyone of claims 1 to 4, wherein the natural ingredients are sealed between the first side and the second side.

6. The skin patch of anyone of claims 1 to 5, further comprising an adhesive on a part of the second side for joining the patch to the skin.

7. The skin patch of anyone of claims 1 to 6, further comprising means for joining the skin patch to the skin with the second side, wherein the means are provided at the first side.

8. The skin patch of anyone of claims 1 to 7, wherein the at least one additional natural ingredient is prepared from extracting a plant or naturally-occurring substance containing the ingredient with an extraction solvent and is contained in the patch in its solid form or as liquid extract being supported on a solid support structure.

9. The skin patch of anyone of claims 1 to 8, wherein the granules having a porous surface have a pore diameter equivalent of 5 to 25 nm.

10. The skin patch of anyone of claims 1 to 9, wherein the granules have a shape in the form of a capsule or a cigar with a diameter r and a length a.

11. The skin patch of anyone of claims 1 to 10, wherein the skin patch is adapted to be joined to the skin of a bottom of a foot.

**EP 2 908 807 B1**

**Patentansprüche**

1. Hautpflaster zum Absorbieren von Giftstoffen aus einem Körper, umfassend
   eine erste Seite;
   eine zweite Seite, die eine Oberfläche umfasst, die zum Absorbieren und Zurückhalten der Giftstoffe angepasst ist, wobei die Oberfläche mehrere Öffnungen, die zur Verstärkung der Übertragung einer Emission von ferner Infrarotstrahlung in die Haut angepasst ist, wenn die zweite Seite in Kontakt mit der Haut kommt; und
   eine granulatförmige bzw. körnige Mischung aus natürlichen Inhaltsstoffen, wobei wenigstens ein Teil der natürlichen Inhaltsstoffe als Granalien bzw. Körner mit einer porösen Oberfläche ausgebildet sind und die Mischung Holzessig, Dextrin und wenigstens einen zusätzlichen Inhaltsstoff umfasst, ausgewählt aus der Gruppe, bestehend aus: Bambusessig, Chitosan, japanische Mispelblätter (Loquat), Houttuynia cordata, Vitamin C, Turmalin, pflanzliche Fasern, Sporopollenin, Pilz-Chitosan, Pfefferminze, Löwenzahn, Schneeball (Viburnum), Fenchel, Süßholz, Anethole, Glycyrrhizinsäure, Capsaicin, Beta-Glucane, Pektin, Antocyane, Champignons, Grapefruit, Zitruspflanzen, Heidelbeeren (Vaccinum), Himbeeren (Rubus-Früchte), Lavendel, Mariendistel (milk thistle), Grüner Tee, Epigallocatechingallat, und L-Theanin, Zimt, Wasabi, Japanischer Ginseng, Japanischer Seetang, Glucomannan, Aloe Vera, Zingerone, Shogaol, Ingwer, Gingerol, Arnika, Nattokinase, Winterkirsche bzw. Lampionblume (winter cherry), Beta-Elemene, Glutathion, Indisches Basilikum (holy basil), Eukalyptus, Amethyst, Kieselgur bzw. Diatomeenerde und Zeolithe.

2. Das Hautpflaster gemäß Anspruch 1, wobei die erste Seite eine Wärme reflektierende Folie und/oder eine nichtgewebte Viskoseoberfläche umfasst, welche optional mit einer Wärme reflektierenden Folie abgedeckt ist.

3. Das Hautpflaster gemäß Anspruch 1 oder Anspruch 2, wobei die Wärme reflektierende Folie eine Metallfolie umfasst, die Kohlenstoffteilchen in ihrer Folienstruktur und/oder verteilt auf der Folienoberfläche umfasst.

4. Das Hautpflaster gemäß einem der Ansprüche 1 bis 3, wobei die Folie ein magnetisches Material oder eine Anzahl von magnetischen Kernen, geträgert auf einer Folie oder komprimiert zwischen zwei Folien, umfasst.

5. Das Hautpflaster gemäß einem der Ansprüche 1 bis 4, wobei die natürliche Inhaltsstoffe zwischen der ersten Seite und der zweiten Seite eingeschlossen sind.

6. Das Hautpflaster gemäß einem der Ansprüche 1 bis 5, das ferner ein Haftmittel auf einem Teil der zweiten Seite zum Anbringen des Pflasters auf der Haut umfasst.

7. Das Hautpflaster gemäß einem der Ansprüche 1 bis 6, das ferner Mittel zum Anbringen des Hautpflasters mit der zweiten Seite auf der Haut umfasst, wobei die Mittel auf der ersten Seite vorgesehen sind.

8. Das Hautpflaster gemäß einem der Ansprüche 1 bis 7, wobei der wenigstens eine zusätzliche natürliche Inhaltsstoff durch Extrahieren einer Pflanze oder einer natürlich vorkommenden Substanz mit dem Inhaltsstoff mit einem Extraktions-Lösungsmittel hergestellt wird und in dem Pflaster in fester Form oder als auf einer festen Trägerstruktur geträgerter flüssiger Extrakt enthalten ist.

9. Das Hautpflaster gemäß einem der Ansprüche 1 bis 8, wobei die Granalien bzw. Körner mit einer porösen Oberfläche einen Porendurchmesseräquivalent von 5 bis 25 nm haben.

10. Das Hautpflaster gemäß einem der Ansprüche 1 bis 9, wobei die Granalien bzw. Körner eine Gestalt in Form einer Kapsel oder einer Zigarre mit einem Durchmesser r und einer Länge a haben.

11. Das Hautpflaster gemäß einem der Ansprüche 1 bis 10, wobei das Hautpflaster derart angepasst ist, dass es auf der Haut einer Fußsohle aufgebracht werden kann.

**Revendications**

1. Timbre transdermique pour absorber des toxines à partir d'un corps, comprenant :

   un premier côté ;
   un second côté comprenant une surface apte à absorber et retenir les toxines, la surface comprenant une pluralité d'ouvertures aptes à améliorer la transmission de l'émission de rayonnement infrarouge lointain dans une peau lorsque le second côté est placé en contact avec la peau ; et

un mélange granulaire d'ingrédients naturels, au moins une partie des ingrédients naturels étant formée en tant que granules ayant une surface poreuse et le mélange comprenant du vinaigre de bois, de la dextrine et au moins un ingrédient supplémentaire choisi dans le groupe consistant en :

vinaigre de bambou, chitosan, feuille de néflier du Japon, houttuynia cordata, vitamine C, tourmaline, fibre végétale, sporopollénine, chitosan fongique, menthe poivrée, pissenlit, blackhaw, fenouil, réglisse, anéthol, acide glycyrrhizique, capsaïcine, bêta-glucanes, pectine, anthocyanines, agaricus, pamplemousse, citrus, baies de Vaccinium, baies de Rubus, lavande, Chardon-Marie, thé vert, gallate d'épigallocatéchine et L-théanine, cannelle, wasabi, ginseng japonais, algue du Japon, glucomannan, aloe vera, zingérone, shogaol, gingembre, gingérol, arnica, nattokinase, cerise d'hiver, bêta-élémène, glutathion, basilic sacré, eucalyptus, améthyste, terre à diatomées et zéolite.

**2.** Timbre transdermique selon la revendication 1, dans lequel le premier côté comprend une feuille réfléchissant la chaleur et/ou une surface de rayonne non tissée, qui est facultativement recouverte d'une feuille réfléchissant la chaleur.

**3.** Timbre transdermique selon la revendication 1 ou la revendication 2, dans lequel la feuille réfléchissant la chaleur comprend une feuille métallique comprenant des particules de carbone dans sa structure de feuille et/ou parsemées sur la surface de feuille.

**4.** Timbre transdermique selon l'une quelconque des revendications 1 à 3, dans lequel la feuille comprend une matière magnétique ou un nombre de noyaux magnétiques supportés sur une feuille ou comprimés entre deux feuilles.

**5.** Timbre transdermique selon l'une quelconque des revendications 1 à 4, dans lequel les ingrédients naturels sont scellés de manière étanche entre le premier côté et le second côté.

**6.** Timbre transdermique selon l'une quelconque des revendications 1 à 5, comprenant en outre un adhésif sur une partie du second côté pour réunir le timbre à la peau.

**7.** Timbre transdermique selon l'une quelconque des revendications 1 à 6, comprenant en outre des moyens pour réunir le timbre transdermique à la peau avec le second côté, les moyens étant disposés sur le premier côté.

**8.** Timbre transdermique selon l'une quelconque des revendications 1 à 7, dans lequel le ou les ingrédients naturels supplémentaires sont préparés par extraction d'une plante ou d'une substance d'origine naturelle contenant l'ingrédient par un solvant d'extraction et sont contenus dans le timbre sous leur forme solide ou sous la forme d'un extrait liquide qui est supporté sur une structure de support solide.

**9.** Timbre transdermique selon l'une quelconque des revendications 1 à 8, dans lequel les granules ayant une surface poreuse ont un diamètre de pore équivalent de 5 à 25 nm.

**10.** Timbre transdermique selon l'une quelconque des revendications 1 à 9, dans lequel les granules ont une configuration en forme de capsule ou de cigare ayant un diamètre r et une longueur a.

**11.** Timbre transdermique selon l'une quelconque des revendications 1 à 10, le timbre transdermique étant apte à être réuni à la peau d'un dessous d'un pied.

100

103

Figure 1A

100

105

107

Figure 1B

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 6610082 B
- US 20100121297 A1
- US 20110064787 A1